# EUROPEAN PATENT APPLICATION

(11) **EP 4 800 004 A1**
(43) Date of publication of application: **02.09.2026**
(21) Application number: 25789235.6
(22) Date of filing: 28.02.2025
(51) Int. Cl.: C07D 207/14, A61K 31/402, A61P 13/12, A61P 35/00, A61P 5/20, A61P 3/14

(54) **ARYLALKYLAMINE COMPOUND, AND PREPARATION METHOD THEREFOR AND USE THEREOF**

(30) Priority: 18.04.2024 CN 202410467711
(71) Applicant: Renhe Yikang Group Co., Ltd., Shijiazhuang, Hebei 051530 (CN)
(72) Inventor: YAN, Hongyi, Shijiazhuang, Hebei 051530 (CN); ZHANG, Kai, Shijiazhuang, Hebei 051530 (CN); NIU, Changqun, Shijiazhuang, Hebei 051530 (CN); SUN, Xiaoyan, Shijiazhuang, Hebei 051530 (CN); DONG, Zongyue, Shijiazhuang, Hebei 051530 (CN); FU, Xiangjie, Shijiazhuang, Hebei 051530 (CN); LIU, Yumiao, Shijiazhuang, Hebei 051530 (CN); ZHANG, Haonan, Shijiazhuang, Hebei 051530 (CN); SHI, Yunrui, Shijiazhuang, Hebei 051530 (CN); ZHANG, Jie, Shijiazhuang, Hebei 051530 (CN); SUN, Huilei, Shijiazhuang, Hebei 051530 (CN); JIA, Qi, Shijiazhuang, Hebei 051530 (CN)
(74) Representative: Isarpatent
(86) International application number: PCT/CN2025/079912
(87) International publication number: WO 2025/218365

(57) **Abstract**

The present invention relates to an arylalkylamine compound, and a preparation method therefor and a use thereof. The arylalkylamine compound of the present invention has a structure represented by formula (I), wherein R¹, R², R³, R⁴, and R⁵ are as defined in the description. The compound of the present invention exhibits excellent calcium-sensing receptor (CaSR) agonistic activity, high bioavailability, high metabolic stability in vivo, and superior safety.

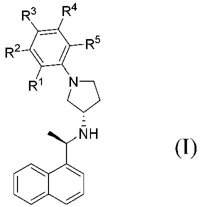

## Description

### FIELD OF THE INVENTION

The invention relates to the field of pharmaceutical technology, and particularly to an arylalkylamine compound, and a preparation method therefor, and a use as an allosteric modulator of the calcium-sensing receptor (CaSR).

### BACKGROUND OF THE INVENTION

Chronic kidney disease (CKD) can lead to secondary hyperparathyroidism (SHPT). Currently, various treatments for SHPT are available worldwide. However, these treatments are associated with several limitations, including high cost, a narrow therapeutic window, incomplete efficacy, susceptibility to relapse, and patient intolerance. Calcimimetics, which act directly on the CaSR and effectively control the synthesis and secretion of parathyroid hormone (PTH), offering significant clinical advantages.

Evocalcet, a calcimimetic drug developed by Mitsubishi Tanabe Pharma Corporation (WO2005115975), was first approved and marketed in Japan in March 2018. According to the review report on Evocalcet, its adverse reactions primarily include hypocalcemia, vomiting, nausea, chills, QT prolongation, mood disturbances, cardiac arrhythmia, hypotension, and cramps (Review Report on Evocalcet Tablets 1 mg and 2 mg, March 2018).

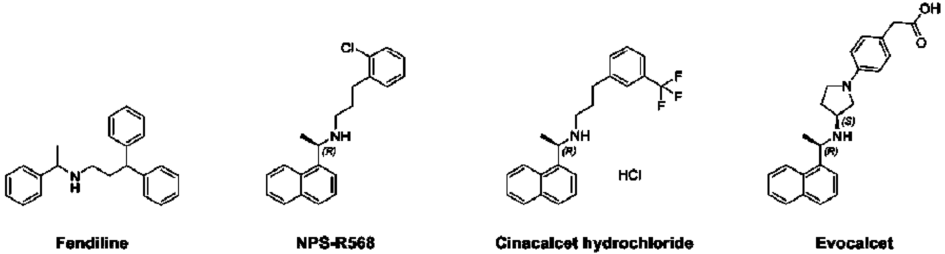

Therefore, the development of calcimimetics, particularly arylalkylamine compounds with higher activity and safety for the treatment of SHPT in patients, holds extremely significant clinical and social value.

### SUMMARY OF THE INVENTION

To address the limitations in the prior art, the invention provides an arylalkylamine compound characterized by enhanced activity and improved safety. The compound of the invention exhibits superior CaSR agonistic activity, high bioavailability, excellent metabolic stability in vivo, and favorable safety. The invention is realized through the following technical solutions.

In one aspect, the invention provides a compound having the structure shown in formula (I), or a pharmaceutically acceptable salt thereof, a stereoisomer thereof, or a tautomer thereof: 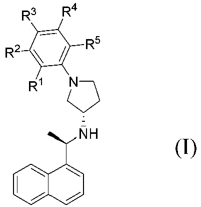
wherein R¹, R², R³, R⁴, and R⁵ are each independently selected from the group consisting of a hydrogen atom; C₂₋₆ carboxyl (-(CH₂)ₙCOOH, n being an integer from 1 to 5); C_{I-7} alkoxy (cycloalkoxy or chain alkoxy), which may be substituted with a substituent selected from halogen, cyano, nitro, oxygen, C₁₋₆ carboxyl (-(CH₂)ₙCOOH, n being an integer from 0 to 5), amine, C₃₋₆ cycloalkyl, aryl C₁₋₆ alkoxy (e.g., benzyloxy), hydroxy, C₁₋₆ alkoxy, halogenated C₁₋₆ alkyl and halogenated C₁₋₆ alkoxy; halogen; C₁₋₆ alkyl; and halogenated C₁₋₆ alkyl;
provided that 1) R¹, R², R³, R⁴, and R⁵ are not simultaneously H; and 2) when the substituent is the C₂₋₆ carboxyl, the substitution site is selected from R²-R⁴, and at least two substituents of R¹-R⁵ are not H.

Preferably, R¹ and R⁵ are each independently selected from H, halogen (e.g. F, Cl), C₁₋₆ alkyl (e.g. methyl, ethyl), C₁₋₆ alkoxy (e.g. methoxy, ethoxy).

Preferably, at least one substituent of R²-R⁴ is an unsubstituted C₂₋₆ carboxyl or a substituted C₂₋₆ carboxyl, preferably carboxymethyl, and at least two substituents of R¹-R⁵ are not H. That is, at least one of the *para* or *meta* sites is carboxymethyl (i.e. -CH₂COOH), and at least one other site is substituted.

Preferably, at least one substituent of R²-R⁴ is an unsubstituted carboxymethyl or a substituted carboxymethyl, and at least two substituents of R¹-R⁵ are not H. That is, at least one of the *para* or *meta* sites is carboxymethyl (i.e. -CH₂COOH), and at least one other site is substituted.

Preferably, R¹, R², R⁴, and R⁵ are each independently selected from H, halogen (e.g. F, Cl), C₁₋₆ alkyl (e.g. methyl, ethyl), and C₁₋₆ alkoxy (e.g. methoxy, ethoxy, etc.) optionally substituted with a substituent selected from hydroxyl, C₁₋₆ alkoxy, and R¹, R², R⁴, and R⁵ are not simultaneously H; and R³ is C₂₋₆ carboxyl.

Preferably, R¹, R², R⁴, and R⁵ are each independently selected from H, halogen (e.g., F, Cl), C₁₋₆ alkyl (e.g. methyl, ethyl), and C₁₋₆ alkoxy (e.g., methoxy, ethoxy, etc.) optionally substituted with a substituent selected from hydroxyl, C₁₋₆ alkoxy, and R¹, R², R⁴ and R⁵ are simultaneously H; and R³ is carboxymethyl.

In a specific embodiment, the compound of formula (I) is selected from:

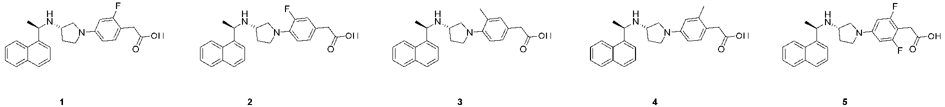

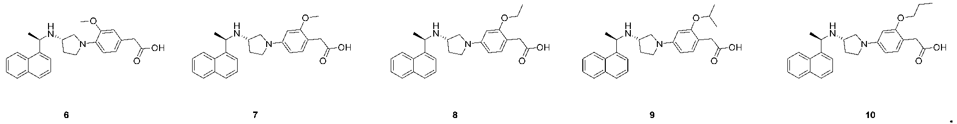

The compound of the general formula (I) of the invention may be in free form or in the form of pharmaceutically acceptable salts. Pharmaceutically acceptable inorganic acid salts include hydrochloride, sulfate, phosphate or hydrobromide salts. Pharmaceutically acceptable organic acid salts include acetate, fumarate, oxalate, citriate, methanesulfonate, benzenesulfonate, p-toluenesulfonate or maleate salts. Additionally, when the compound contains an acidic group such as a carboxyl group, it may form salts with metal ions, such as alkali metal salts (e.g., sodium salts, potassium salts) or alkaline earth metal salts (e.g., calcium salts).

On the other hand, the invention provides a method for preparing the compound of formula (I), which comprises the step of reacting a compound of formula (A) with a compound of formula (II) to form the compound of formula (I):

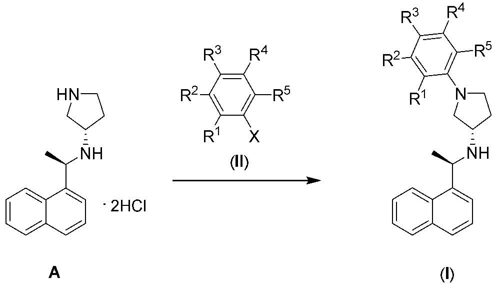

Wherein X represents a leaving group selected from halogen, hydroxyl, lower alkylsulfonyloxy (e.g. C₁₋₆ alkylsulfonyloxy), or sulfonyloxy, preferably selected from Br, Cl, or trifluoromethanesulfonyloxy.

In a further aspect, the invention provides a pharmaceutical composition comprising the above compound or the pharmaceutically acceptable salt thereof, or the stereoisomer thereof or the tautomer thereof and a pharmaceutically acceptable carrier or excipient.

Specifically, the compound of formula (I) of the invention, or the pharmaceutically acceptable salt thereof, when used as an active pharmaceutical ingredient, can be formulated with an inert carrier suitable for each method of administration into conventional pharmaceutical preparations, such as tablets, granules, capsules, powders, solutions, suspensions, emulsions, injections, and the like. When the pharmaceutical preparation is used as a solid preparation, the inert carrier includes a binder (e.g., gum arabic, gelatin, sorbitol, polyvinylpyrrolidone, etc.), an excipient (e.g., lactose, galactose, corn starch, sorbitol, etc.), a lubricant (e.g., magnesium stearate, talc, polyethylene glycol, etc.) and a disintegrant (e.g., potato starch, etc.). When the pharmaceutical preparation is used as an injection solution, it can be prepared with distilled water for injection, normal saline, aqueous glucose solution, and so on.

The compound of formula (I) or the pharmaceutically acceptable salt thereof of the invention may be administered via oral administration, intravenous injection, intramuscular injection, subcutaneous injection, or transdermal absorption routes. The dosage and the volume of administration may be determined based on factors such as the properties of the drug, the route of administration, and the patient's age, the patient's weight, or disease state.

The invention provides the above compound or the pharmaceutically acceptable salt thereof, or the stereoisomer thereof, or the tautomer thereof, or the pharmaceutical composition comprising the above compound or the pharmaceutically acceptable salt thereof, or the stereoisomer thereof, or the tautomer thereof, and a pharmaceutically acceptable carrier or excipient. These compounds produce a variety of pharmacological effects by activating CaSR, and are used for the prevention and treatment of CaSR-related diseases, such as inhibiting the production of PTH and regulating the calcium and phosphorus levels in the blood. They are also used for the treatment of primary hyperparathyroidism, secondary hyperparathyroidism, tertiary hyperparathyroidism, chronic renal failure (with or without dialysis), chronic kidney disease (with or without dialysis), parathyroid adenoma, parathyroid hyperplasia, parathyroid carcinoma, vascular calcification and valvular calcification, abnormal calcium homeostasis (e.g., hypercalcemia), abnormal phosphorus homeostasis (e.g., hypophosphatemia), bone-related disorders or complications due to hyperparathyroidism, chronic kidney disease, or parathyroid carcinoma, bone loss after kidney transplantation, osteitis fibrosa cystica, adynamic bone disease, renal osteodystrophy, cardiovascular complications due to hyperparathyroidism or chronic kidney disease, and certain malignancies with abnormally high Ca²⁺ levels.

Unless otherwise indicated, the following terms used in the description and claims have the meanings provided below.

The invention encompasses all stereoisomeric forms of the compound. Unless a specific stereoisomeric configuration is indicated, the invention is intended to encompass all such isomeric forms. The asymmetric centres in the compound of the invention may each independently have either the (*R*) configuration or the (S) configuration. When a bond connected to a chiral carbon is represented as a straight line in the structural formula, it is understood that both the (*R*) and (*S*) conformations of the chiral carbon, as well as the twoenantiomers and mixture thereof, are included in the structural formula. When a specific configuration is depicted, the intended enantiomer (*R*) or (*S*) is explicitly indicated. Similarly, when a compound name is provided without specifying the chiral configuration, it is understood that both the (*R*) and (*S*) configurations, as well as individual enantiomer and mixture thereof, are encompassed in the compound named. The preparation of specific stereoisomers or mixture thereof may be described in the examples where these stereoisomers or mixture are obtained; however, this does not limit the inclusion of all stereoisomers and mixture within the scope of the invention.

The term "carboxyl" refers to a fundamental functional group in organic chemistry, consisting of one carbon atom, two oxygen atoms, and one hydrogen atom, with the chemical formula-COOH. C₁₋₆ carboxyl and C₂₋₆ carboxyl are lower alkyl groups that additionally contain carboxyl. Examples of C₁₋₆ carboxyl include e.g., -COOH, -CH₂COOH, -CH₂CH₂COOH, -CH₂CH₂CH₂COOH,-CH₂CH₂CH₂CH₂COOH, and -CH₂CH₂CH₂CH₂CH₂COOH. Similarly, C₂₋₆ carboxyl may include-CH₂COOH, -CH₂CH₂COOH, -CH₂CH₂CH₂COOH, -CH₂CH₂CH₂CH₂COOH, and-CH₂CH₂CH₂CH₂CH₂COOH.

The compound of formula (I) of the invention, or the pharmaceutically acceptable salt thereof, acts as an active ingredient that allosterically activates the CaSR on parathyroid cells, thereby inhibiting the synthesis and secretion of PTH. This mechanism reduces PTH levels in the blood, thereby stabilizing blood calcium and phosphorus concentrations. The compound is effective for the prevention or treatment of hyperparathyroidism, demonstrating excellent allosteric agonistic effects on CaSR and significant PTH reduction *in vivo*. The CaSR allosteric agonistic activity and PTH-lowering effects of the compound were confirmed in model rats through CaSR allosteric agonistic experiments, adenine-induced hyperparathyroidism model in SD rats, and 5/6 nephrectomy model in SD rats.

The compound of formula (I) of the invention, or the pharmaceutically acceptable salt thereof, as an active ingredient, not only exhibits excellent pharmacological activity, but also demonstrates significant in vivo stability. Rat liver microsomal incubation experiments were conducted to confirm the metabolic stability of the compound. The compound of formula (I) of the invention, or the pharmaceutically acceptable salt thereof, as an active ingredient, not only exhibits excellent CaSR agonistic activity, but also exhibits a high safety. Compared to the prior art, the invention offers superior activity, safety, in vivo metabolic stability, and relative bioavailability.

### DETAILED DESCRIPTION OF THE INVENTION

The invention is further described below in connection with examples; however, these examples are not intended to limit the scope of the invention.

The known starting materials of the invention can be synthesized using or in accordance with methods known in the art, or can be purchased from chemical companies such as Energy Chemical, J&K Scientific, Macklin, Aladdin, Bide Pharmatech and so on.

In the examples, unless otherwise specified, "solution" refers to an aqueous solution; "room temperature" refers to a temperature ranging from 20°C to 30°C; and "inert gas" refers to argon or nitrogen. The eluent systems used for reaction monitoring by thin-layer chromatography (TLC) and for purification by column chromatography include dichloromethane/methanol, n-hexane/ethyl acetate, petroleum ether/ethyl acetate, and petroleum ether/ethyl acetate/methanol. The ratio of the system components is adjusted according to the polarity of the compound. Additionally, a small amount of alkaline or acidic reagents, such as triethylamine or acetic acid, may be added for further adjustment.

In the examples, unless otherwise specified, structural confirmation was performed using mass spectrometry (MS), nuclear magnetic resonance (NMR), and high performance liquid chromatography (HPLC). The instrument models used were as follows: MS: Agilent InfinityLab LC/MSD iQ G6160A, NMR: Bruker AVANCE 400 or Bruker AVANCE NEO 600, HPLC: Shimadzu LC-2010AHT. For NMR measurements, the following deuterated solvents were used: deuterated dimethyl sulfoxide (DMSO-*d*₆), deuterated chloroform (CDCl₃), deuterated methanol (CD₃OD), or deuterated water (D₂O). Tetramethylsilane (TMS) was used as the internal standard.

### Example 1: Synthesis of (S)-N-((R)-1-(naphthalen-1-yl)ethyl)pyrrolidin-3-amine dihydrochloride (A)

The synthetic route is as follows:

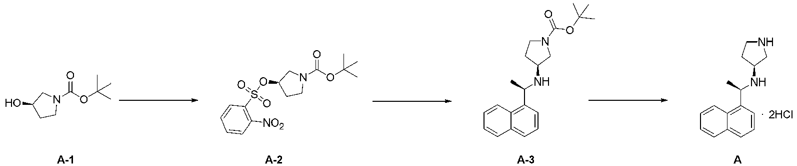

### Step 1: tert-butyl (R)-3-(((2-nitrophenyl)sulfonyl)oxy)pyrrolidine-1-carboxylate (A-2).

To a 500 mL three-necked flask, compound **A-1** (25.00 g, 0.13 mol), triethylamine (17.57 g, 0.17 mol), trimethylamine hydrochloride (2.55 g, 0.03 mol), and dichloromethane (125 mL) were added and stirred. The reaction mixture was cooled to 0°C~10°C, and a solution of 2-nitrobenzenesulfonyl chloride (32.55 g, 0.15 mol) in dichloromethane (125 mL) was added dropwise. After the reaction was completed, 150 mL of purified water was added, and the pH was adjusted to 2.0~4.0 using 6N hydrochloric acid. The reaction mixture was allowed to stand until obvious phase separation occurred. The organic phase and the aqueous phase were separated, and the organic phase was retained. The aqueous phase was extracted once with 50 mL of dichloromethane. The organic phases were combined, dried with anhydrous sodium sulfate and filtered. The filtrate was concentrated under reduced pressure to a constant weight to obtain compound **A-2** (47.40 g, yield = 95%). LCMS (ESI) m/z: 373.1 [M+H]⁺.

### Step 2: tert-butyl (S)-3-(((R)-1-(naphthalen-1-yl)ethyl)amino)pyrrolidine-1-carboxylate (A-3)

To a 100 mL three-necked flask, compound **A-2** (45.00 g, 0.12 mol), (1*R*)-1-(naphthalen-1-yl)ethan-1-amine (17.60 g, 0.10 mol), potassium phosphate (21.23 g, 0.10 mol), and acetonitrile (150 mL) were added. The reaction mixture was heated to 75°C and stirred for 24 h. The reaction mixture was cooled to room temperature, and filtered under vacuum. The filter cake was washed with 60 mL of acetonitrile. The filtrate was concentrated under reduced pressure until no solvent was evaporated. Ethyl acetate (90 mL) and saturated brine (90 mL) were added, and the mixture was stirred for 0.5 h. The mixture was allowed to stand until obvious phase separation occurred, and the organic phase and the aqueous phase were separated. The organic phase was retained, and the aqueous phase was extracted once with 45 mL of ethyl acetate. The organic phases were combined and dried with anhydrous sodium sulfate. The filtrate was concentrated under reduced pressure to a constant weight to obtain compound **A-3** (32.62 g, yield = 96%). LCMS (ESI) m/z: 341.2 [M+H]⁺.

### Step 3: (S)-N-((R)-1-(naphthalen-1-yl)ethyl)pyrrolidin-3-amine dihydrochloride (A)

To a 500 mL three-necked flask, compound **A-3** (30.00 g, 0.09 mol) and ethyl acetate (150 mL) were added and stirred. The reaction mixture was cooled to 0°C~10°C, and concentrated hydrochloric acid (22.5 mL) was added dropwise. After the addition was completed, the reaction mixture was stirred at the same temperature for 0.5 h and heated to 55°C to react for 2.0 h. The reaction mixture was cooled to room temperature and filtered. The filter cake was washed with 50 mL of ethyl acetate and dried under forced air flow at 45°C to a constant weight to obtain compound **A** (19.87 g, yield = 92%). LCMS (ESI) m/z: 241.2 [M+H]⁺. ¹H NMR (400 MHz, MeOH-*d*₄): δ 8.29 (d, *J* = 8.8 Hz, 1H), 8.04-7.97 (m, 2H), 7.94 (d, *J* = 7.2 Hz ,1H), 7.72-7.58 (m, 3H), 5.60-5.51 (m, 1H), 3.98-3.87 (m, 1H), 3.65-3.54 (m, 1H), 3.51-3.38 (m, 2H), 3.29-3.20 (m, 1H), 2.55-2.44 (m, 1H), 2.43-2.32 (m, 1H), 1.89 (d, *J* = 6.8 Hz, 3H).

### Example 2: Synthesis of 2-(2-fluoro-4-((S)-3-(((R)-1-(naphthalen-1-yl)ethyl)amino)pyrrolidin-1-yl)phenyl)acetic acid (1).

The synthetic route is as follows:

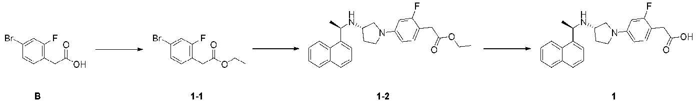

### Step 1: ethyl 2-(4-bromo-2-fluorophenyl)acetate (1-1)

To a 250 mL single-necked flask, compound **B** (2.00 g, 8.58 mmol) and ethanol (60 mL) were added and stirred. The reaction mixture was cooled to 0°C, and concentrated sulfuric acid (1 mL) was added dropwise. The reaction mixture was heated to reflux for 3.0 h. The reaction mixture was cooled to room temperature, and concentrated under reduced pressure until no solvent was evaporated. Ethyl acetate (50 mL) and purified water (50 mL) were added and stirred. A saturated sodium carbonate solution was added dropwise to adjust the pH to 8.0. The mixture was allowed to stand until obvious phase separation occurred, and the organic phase and the aqueous phase were separated. The organic phase was retained, and the aqueous phase was extracted once with 50 mL of ethyl acetate. The organic phases were combined and dried with anhydrous sodium sulfate, followed by filtration. The filtrate was concentrated under reduced pressure to a constant weight to obtain compound **1-1** (2.00 g, yield = 89%). LCMS (ESI) m/z: 261.0 [M+H]⁺.

### Step 2: ethyl 2-(2-fluoro-4-((S)-3-(((R)-1-(naphthalen-1-yl)ethyl)amino)pyrrolidin-1-yl)phenyl)acetate (1-2)

To a 250 mL single-necked flask, free compound **A** (1.00 g, 4.16 mmol), compound **1-1** (1.31 g, 4.99 mmol), Pd(OAc)₂ (50 mg, 0.20 mmol), X-Phos (100 mg, 0.20 mmol), Cs₂CO₃ (4.10 g, 12.48 mmol), and toluene (50 mL) were added. The system was purged with nitrogen three times, and the reaction mixture was heated to 100°C and stirred for 12.0 h. The reaction mixture was cooled to room temperature, and 30 mL of saturated brine was added and stirred. The mixture was allowed to stand until obvious phase separation occurred. The organic phase and the aqueous phase were separated, and the organic phase was retained. The aqueous phase was extracted once with 20 mL of ethyl acetate. The organic phases were combined, dried with anhydrous sodium sulfate, and filtered. The filtrate was concentrated under reduced pressure until no solvent was evaporated. The residue was purified by column chromatography (200-300 mesh silica gel, petroleum ether/ethyl acetate (volume ratio of petroleum ether to ethyl acetate): 80/20 → 50/50). The purified product was concentrated under reduced pressure to obtain compound **1-2** (0.70 g, yield = 40%). LCMS (ESI) m/z: 421.2 [M+H]⁺.

### Step 3: 2-(2-fluoro-4-((S)-3-(((R)-1-(naphthalen-1-yl)ethyl)amino)pyrrolidin-1-yl)phenyl)acetic acid (1)

To a 100 mL single-necked flask, compound **1-2** (0.60 g, 1.43 mmol), sodium hydroxide (572 mg, 14.3 mmol), purified water (10 mL), and ethanol (10 mL) were added. The reaction mixture was stirred at 60°C for 3.0 h and cooled to room temperature. The pH was adjusted with 1N hydrochloric acid until solid was visibly precipitated. The mixture was filtered, and the filter cake was washed with 10 mL of purified water. The solid was dried under forced air flow at 45°C to a constant weight to obtain compound **1** (0.30 g, yield = 53%). LCMS (ESI) m/z: 393.3 [M+H]⁺. ¹H NMR (600 MHz, DMSO-*d*₆) δ 8.34 (d, *J* = 8.4 Hz, 1H), 7.96 (d, *J* = 7.9 Hz, 1H), 7.87 (d, *J* = 8.6 Hz, 2H), 7.56 (dd, *J* = 14.2, 5.9 Hz, 3H), 7.05 (t, *J* = 8.6 Hz, 1H), 6.24 (d, *J* = 8.9 Hz, 2H), 4.96 (s, 1H), 3.43 (s, 3H), 3.36 (q, *J* = 7.7 Hz, 2H), 3.13 (q, *J* = 7.8 Hz, 2H), 2.07 (m, 2H), 1.52 (s, 3H).

### Example 3: Synthesis of 2-(3-fluoro-4-((S)-3-(((R)-1-(naphthalen-1-yl)ethyl)amino)pyrrolidin-1-yl)phenyl)acetic acid (2)

The synthetic route is as follows:

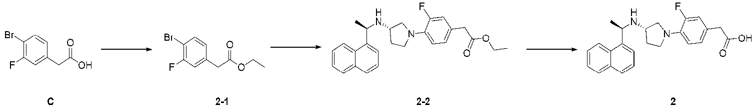

### Step 1: ethyl 2-(4-bromo-3-fluorophenyl)acetate (2-1)

To a 250 mL single-necked flask, compound **C** (2.00 g, 8.58 mmol) and ethanol (60 mL) were added and stirred. The reaction mixture was cooled to 0°C, and concentrated sulfuric acid (1 mL) was added dropwise. The synthesis and work-up procedures were carried out following the method described for compound **1-1**, and compound **2-1** (1.80 g, yield = 81%) was obtained. LCMS (ESI) m/z: 261.0 [M+H]⁺.

### Step 2: ethyl 2-(3-fluoro-4-((S)-3-(((R)-1-(naphthalen-1-yl)ethyl)amino)pyrrolidin-1-yl)phenyl)acetate (2-2)

To a 250 mL single-necked flask, the free compound **A** (1.00 g, 4.16 mmol), compound **2-1** (1.31 g, 4.99 mmol), Pd(OAc)₂ (50 mg, 0.20 mmol), X-Phos (100 mg, 0.20 mmol), Cs₂CO₃ (4.10 g, 12.48 mmol), and toluene (50 mL) were added. The synthesis and work-up procedures were performed following the method described for compound **1-2**. Compound **2-2** (0.89 g, yield = 51%) was obtained. LCMS (ESI) m/z: 421.2 [M+H]⁺.

### Step 3: 2-(3-fluoro-4-((S)-3-(((R)-1-(naphthalen-1-yl)ethyl)amino)pyrrolidin-1-yl)phenyl)acetic acid (2)

To a 100 mL single-necked flask, compound **2-2** (0.80 g, 1.90 mmol), sodium hydroxide (760 mg, 19.0 mmol), purified water (10 mL), and ethanol (10 mL) were added. The synthesis and work-up procedures were performed following the method described for compound **1**, yielding compound **2** (0.39 g, yield = 52%). LCMS (ESI) m/z: 393.3 [M+H]⁺. ¹H NMR (400 MHz, DMSO-*d*₆) δ 8.33-8.26 (m, 1H), 7.93 (dd, *J =* 7.0, 2.3 Hz, 1H), 7.79 (d, *J =* 8.1 Hz, 1H), 7.72 (d, *J =* 7.2 Hz, 1H), 7.51 (dtd, *J =* 10.2, 7.2, 4.8 Hz, 3H), 6.91 (dd, *J =* 14.9, 2.0 Hz, 1H), 6.85 (dd, *J =* 8.2, 2.0 Hz, 1H), 6.56 (t, *J =* 9.0 Hz, 1H), 4.72 (q, *J =* 6.6 Hz, 1H), 3.42-3.28 (m, 4H), 3.19 (p, *J =* 6.9, 5.9 Hz, 2H), 3.09 (ddd, *J =* 8.9, 5.7, 2.7 Hz, 1H), 1.96 (dq, *J* = 12.3, 6.1 Hz, 1H), 1.83 (dq, *J* = 13.6, 7.2 Hz, 1H), 1.40 (d, *J* = 6.5 Hz, 3H).

### Example 4: Synthesis of 2-(3-methyl-4-((S)-3-(((R)-1-(naphthalen-1-yl)ethyl)amino)pyrrolidin-1-yl)phenyl)acetic acid (3)

The synthetic route is as follows:

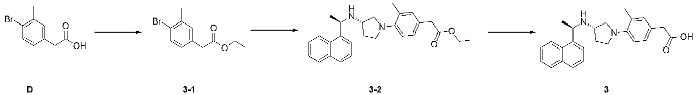

### Step 1: ethyl 2-(4-bromo-3-methylphenyl)acetate (3-1)

To a 250 mL single-necked flask, compound **D** (1.00 g, 4.37 mmol) and ethanol (60 mL) were added and stirred. The reaction mixture was cooled to 0°C, and concentrated sulfuric acid (1 mL) was added dropwise. The synthesis and work-up procedures were performed following the method described for compound **1-1**, yielding compound **3-1** (0.87 g, yield = 77%). LCMS (ESI) m/z: 257.0 [M+H]⁺.

### Step 2: ethyl 2-(3-methyl-4-((S)-3-(((R)-1-(naphthalen-1-yl)ethyl)amino)pyrrolidin-1-yl)phenyl)acetate (3-2)

To a 250 mL single-necked flask, compound A in free form (1.00 g, 4.16 mmol), compound **3-1** (1.28 g, 4.99 mmol), Pd(OAc)₂ (50 mg, 0.20 mmol), X-Phos (100 mg, 0.20 mmol), Cs₂CO₃ (4.10 g, 12.48 mmol), and toluene (50 mL) were added. The synthesis and work-up procedures were carried out following the method described for compound **1-2**, and compound **3-2** (0.73 g, yield = 42%) was obtained. LCMS (ESI) m/z: 417.2 [M+H]⁺.

### Step 3: 2-(3-methyl-4-((S)-3-(((R)-1-(naphthalen-1-yl)ethyl)amino)pyrrolidin-1-yl)phenyl)acetic acid (3)

To a 100 mL single-necked flask, compound **3-2** (0.70 g, 1.68 mmol), sodium hydroxide (0.34 g, 8.40 mmol), purified water (10 mL), and ethanol (10 mL) were added. The synthesis and work-up procedures were carried out with reference to compound **1**, and compound **3** (0.44 g, yield = 67%) was obtained. LCMS (ESI) m/z: 389.2 [M+H]⁺. ¹H NMR (400 MHz, DMSO-*d*₆) δ 8.32 (d, *J* = 8.0 Hz, 1H), 7.93 (dd, *J* = 7.3, 2.1 Hz, 1H), 7.80 (d, *J* = 8.1 Hz, 1H), 7.73 (d, *J* = 7.1 Hz, 1H), 7.51 (qd, *J* = 8.1, 7.3, 2.3 Hz, 3H), 6.97-6.86 (m, 2H), 6.71-6.64 (m, 1H), 4.71 (q, *J* = 6.5 Hz, 1H), 3.38 (s, 2H), 3.18 (s, 1H), 3.13-3.06 (m, 2H), 3.02 (m, 1H), 2.92 (dd, *J* = 9.2, 6.3 Hz, 1H), 2.11 (s, 3H), 1.99 (dq, *J* = 12.6, 6.5 Hz, 1H), 1.86-1.73 (m, 1H), 1.41 (d, *J* = 6.5 Hz, 3H).

### Example 5: Synthesis of 2-(2-methyl-4-((S)-3-(((R)-1-(naphthalen-1-yl)ethyl)amino)pyrrolidin-1-yl)phenyl)acetic acid (4)

The synthetic route is as follows:

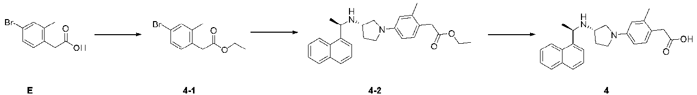

### Step 1: ethyl 2-(4-bromo-2-methylphenyl)acetate (4-1)

To a 250 mL single-necked flask, compound **E** (1.00 g, 4.37 mmol) and ethanol (60 mL) were added and stirred. The reaction mixture was cooled to 0°C, and concentrated sulfuric acid (1 mL) was added dropwise. The synthesis and work-up procedures were carried out following the method described for compound **1-1**, and compound **4-1** (0.93 g, yield = 83%) was obtained. LCMS (ESI) m/z: 257.0 [M+H]⁺.

### Step 2: ethyl 2-(2-methyl-4-((S)-3-(((R)-1-(naphthalen-1-yl)ethyl)amino)pyrrolidin-1-yl)phenyl)acetate (4-2)

To a 250 mL single-necked flask, the free compound **A** (1.00 g, 4.16 mmol), compound **4-1** (1.28 g, 4.99 mmol), Pd(OAc)₂ (50 mg, 0.20 mmol), X-Phos (100 mg, 0.20 mmol), Cs₂CO₃ (4.10 g, 12.48 mmol), and toluene (50 mL) were added. The synthesis and work-up procedures were performed following the method described for compound **1-2**, and compound **4-2** (0.73 g, yield = 42%) was obtained. LCMS (ESI) m/z: 417.2 [M+H]⁺.

### Step 3: 2-(2-methyl-4-((S)-3-(((R)-1-(naphthalen-1-yl)ethyl)amino)pyrrolidin-1-yl)phenyl)acetic acid (4)

To a 100 mL single-necked flask, compound **4-2** (0.50 g, 1.20 mmol), sodium hydroxide (0.24 g, 6.00 mmol), purified water (5 mL), and ethanol (5 mL) were added. The synthesis and work-up procedures were performed following the method described for compound **1**, and compound **4** (0.32 g, yield = 69%) was obtained. LCMS (ESI) m/z: 389.2 [M+H]⁺. ¹H NMR (400 MHz, DMSO-*d*₆) δ 8.30 (d, *J* = 8.0 Hz, 1H), 7.92 (dd, *J* = 7.3, 2.1 Hz, 1H), 7.81 (d, *J* = 8.1 Hz, 1H), 7.73 (d, *J* = 7.1 Hz, 1H), 7.53 (qd, *J* = 8.1, 7.3, 2.3 Hz, 3H), 6.97-6.86 (m, 2H), 6.71-6.64 (m, 1H), 4.71 (q, *J* = 6.5 Hz, 1H), 3.38 (s, 2H), 3.18 (s, 1H), 3.13-3.06 (m, 2H), 3.02 (m, 1H), 2.91 (dd, *J* = 9.2, 6.3 Hz, 1H), 2.10 (s, 3H), 1.99 (dq, *J* = 12.6, 6.5 Hz, 1H), 1.86-1.72 (m, 1H), 1.43 (d, *J* = 6.5 Hz, 3H).

### Example 6: Synthesis of 2-(2,6-difluoro-4-((S)-3-(((R)-1-(naphthalen-1-yl)ethyl)amino)pyrrolidin-1-yl)phenyl)acetic acid (5)

The synthetic route is as follows:

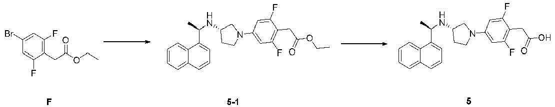

### Step 1: ethyl 2-(2,6-difluoro-4-((S)-3-(((R)-1-(naphthalen-1-yl)ethyl)amino)pyrrolidin-1-yl)phenyl)acetate (5-1)

To a 250 mL single-necked flask, free compound **A** (861 mg, 3.58 mmol), compound **F** (1.00 g, 3.58 mmol), Pd(OAc)₂ (80 mg, 0.36 mmol), X-Phos (171 mg, 0.36 mmol), Cs₂CO₃ (3.50 g, 10.74 mmol), and toluene (50 mL) were added. The synthesis and work-up procedures were performed following the method described for compound **1-2**, and compound **5-1** (1.44 g, yield = 74%) was obtained. LCMS (ESI) m/z: 439.2 [M+H]⁺.

### Step 2: 2-(2,6-difluoro-4-((S)-3-(((R)-1-(naphthalen-1-yl)ethyl)amino)pyrrolidin-1-yl)phenyl)acetic acid (5)

To a 100 mL single-necked flask, compound **5-1** (1.44 g, 3.28 mmol), sodium hydroxide (0.66 g, 16.43 mmol), purified water (5 mL), and ethanol (5 mL) were added. The synthesis and work-up procedures were performed following the method described for compound **1**. Compound **5** (1.25 g, yield = 93%) was obtained. LCMS (ESI) m/z: 411.2 [M+H]⁺. ¹H NMR (600 MHz, DMSO-*d*₆) δ 8.30 (s, 1H), 7.94 (s, 1H), 7.85-7.78 (m, 1H), 7.73 (dd, *J* = 10.9, 6.0 Hz, 1H), 7.57-7.47 (m, *J* = 6.5, 5.5 Hz, 3H), 6.08 (dq, *J* = 10.2, 5.2 Hz, 2H), 4.75 (dd, *J* = 11.7, 6.0 Hz, 1H), 3.42 (q, *J* = 5.5, 5.0 Hz, 2H), 3.37-3.23 (m, 3H), 3.11 (s, 1H), 2.99 (s, 1H), 2.05-1.98 (m, 1H), 1.94-1.88 (m, 1H), 1.42 (p, *J* = 5.7 Hz, 3H).

### Example 7: Synthesis of 2-(3-methoxy-4-((S)-3-(((R)-1-(naphthalen-1-yl)ethyl)amino)pyrrolidin-1-yl)phenyl)acetic acid (6)

The synthetic route is as follows:

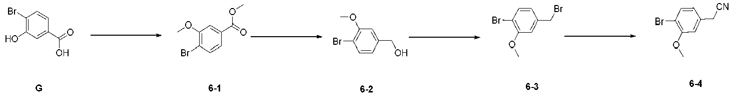

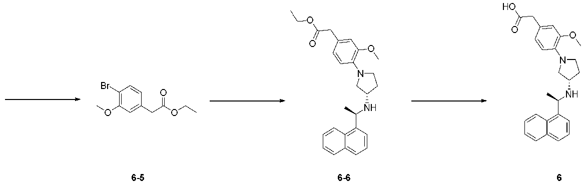

### Step 1: methyl 4-bromo-3-methoxybenzoate (6-1)

To a 100 mL single-necked flask, the free compound G (10.00 g, 0.046 mol), methyl iodide (19.62 g, 0.14 mol), potassium carbonate (25.47 g, 0.18 mol), and 50 mL of DMF were added and stirred overnight. Saturated brine (500 mL) and ethyl acetate (100 mL) were added, and the mixture was stirred. The mixture was allowed to stand until obvious phase separation occurred, and the organic phase and the aqueous phase were separated. The organic phase was retained, and the aqueous phase was extracted twice with 50 mL of ethyl acetate. The organic phases were combined and dried with anhydrous sodium sulfate. The mixture was filtered, and the filtrate was concentrated under reduced pressure until no solvent was evaporated to obtain compound **6-1** (10.50 g, yield = 93%). LCMS (ESI) m/z: 245.0 [M+H]⁺.

### Step 2: (4-bromo-3-methoxyphenyl)methanol (6-2)

To a 500 mL three-necked flask, compound **6-1** (10.50 g, 23.8 mmol) and 100 mL of anhydrous tetrahydrofuran were added and stirred. The reaction mixture was cooled to 0°C, and lithium aluminum hydride (1.95 g, 51.4 mmol) was added portionwise. The reaction was maintained at this temperature for 4.0 h. 150 mL of 1N hydrochloric acid was slowly added, and the mixture was stirred. The mixture was allowed to stand until obvious phase separation occurred, and the organic phase and the aqueous phase were separated. The organic phase was retained, and the aqueous phase was extracted twice with 50 mL of ethyl acetate. The organic phases were combined and dried with anhydrous sodium sulfate. The mixture was filtered, and the filtrate was concentrated under reduced pressure until no solvent was evaporated. The residue was purified by column chromatography (200-300 mesh silica gel, petroleum ether/ethyl acetate: 95/5→80/20). The purified product was concentrated under reduced pressure to obtain compound **6-2** (5.80 g, yield = 62%). LCMS (ESI) m/z: 217.0 [M+H]⁺.

### Step 3: 1-bromo-4-(bromomethyl)-2-methoxybenzene (6-3)

To a 500 mL three-necked flask, compound **6-2** (5.00 g, 23.00 mmol), triphenylphosphine (7.30 g, 27.60 mmol), carbon tetrabromide (8.40 g, 25.30 mmol), and dichloromethane (100 mL) were added and stirred overnight. The filtrate was concentrated under reduced pressure until no solvent was evaporated. The residue was purified by column chromatography (200-300 mesh silica gel, petroleum ether/ethyl acetate: 95/5 → 85/15). The purified product was concentrated under reduced pressure to obtain compound **6-3** (5.80 g, yield = 90%). LCMS (ESI) m/z: 279.0 [M+H]⁺.

### Step 4: 2-(4-bromo-3-methoxyphenyl)acetonitrile (6-4)

To a 500 mL three-necked flask, compound **6-3** (5.00 g, 17.9 mmol), tetrabutylammonium fluoride (TBAF) (5.60 g, 21.4 mmol), and acetonitrile (50 mL) were added and stirred. The mixture was cooled to 0°C, and trimethylsilyl cyanide (TMSCN) (2.13 g, 21.4 mmol) was added portionwise. The reaction was stirred overnight, and the filtrate was concentrated under reduced pressure until no solvent was evaporated. The residue was purified by column chromatography (200-300 mesh silica gel, petroleum ether/ethyl acetate: 95/5 → 85/15). The purified product was concentrated under reduced pressure to obtain compound **6-4** (3.60 g, yield = 89%). LCMS (ESI) m/z: 226.0 [M+H]⁺.

### Step 5: ethyl 2-(4-bromo-3-methoxyphenyl)acetate (6-5)

To a 500 mL three-necked flask, compound **6-4** (3.00 g, 13.27 mmol) and 50 mL of ethanol were added and stirred. The reaction mixture was cooled to 0°C, and concentrated sulfuric acid (7.5 mL) was added dropwise. The synthesis and work-up procedures were carried out following the method described for compound **1-1**. Compound **6-5** (3.20 g, yield = 88%) was obtained. LCMS (ESI) m/z: 273.0 [M+H]⁺.

### Step 6: ethyl 2-(3-methoxy-4-((S)-3-(((R)-1-(naphthalen-1-yl)ethyl)amino)pyrrolidin-1-yl)phenyl)acetate (6-6)

To a 100 mL single-necked flask, free compound **A** (1.00 g, 4.61 mmol), compound **6-5** (1.10 g, 4.99 mmol), Pd(OAc)₂ (19 mg, 0.08 mmol), X-Phos (40 mg, 0.08 mmol), Cs₂CO₃ (4.00 g, 12.50 mmol), and toluene (20 mL) were added. The synthesis and work-up procedures were carried out following the method described for compound **1-2**, and compound **6-6** (1.20 g, yield = 67%) was obtained. LCMS (ESI) m/z: 433.2 [M+H]⁺.

### Step 7: 2-(3-methoxy-4-((S)-3-(((R)-1-(naphthalen-1-yl)ethyl)amino)pyrrolidin-1-yl)phenyl)acetic acid (6)

To a 100 mL single-necked flask, compound **6-6** (1.00 g, 2.31 mmol), sodium hydroxide (0.28 g, 6.93 mmol), purified water (5 mL), and ethanol (5 mL) were added. The synthesis and work-up procedures were carried out following the method described for compound **1**. Compound **6** (0.31 g, yield = 33%) was obtained. LCMS (ESI) m/z: 405.2 [M+H]⁺. ¹H NMR (600 MHz, DMSO-*d*₆) δ 8.32 (d, *J* = 8.2 Hz, 1H), 7.92 (dd, *J* = 7.7, 1.8 Hz, 1H), 7.79 (d, *J* = 8.2 Hz, 1H), 7.72 (dd, *J* = 7.3, 1.2 Hz, 1H), 7.55-7.46 (m, 3H), 6.73 (d, *J* = 2.0 Hz, 1H), 6.65 (dd, *J* = 8.1, 1.9 Hz, 1H), 6.48 (d, *J* = 8.1 Hz, 1H), 4.71 (s, 1H), 3.64 (s, 3H), 3.40 (s, 2H), 3.22 (dt, *J* = 7.9, 6.0 Hz, 2H), 3.14 (dt, *J* = 9.4, 6.8 Hz, 2H), 3.01 (t, *J* = 7.9 Hz, 1H), 1.93 (dt, *J* = 13.2, 6.6 Hz, 1H), 1.77 (dd, *J* = 12.5, 6.7 Hz, 1H), 1.40 (d, *J* = 6.6 Hz, 3H).

### Example 8: Synthesis of 2-(2-methoxy-4-((S)-3-(((R)-1-(naphthalen-1-yl)ethyl)amino)pyrrolidin-1-yl)phenyl)acetic acid (7)

The synthetic route is as follows:

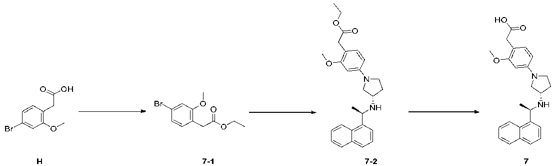

### Step 1: ethyl 2-(4-bromo-2-methoxyphenyl)acetate (7-1)

To a 500 mL three-necked flask, compound **H** (10.00 g, 40.80 mmol) and ethanol (80 mL) were added and stirred. The reaction mixture was cooled to 0°C, and concentrated sulfuric acid (1.5 mL) was added dropwise. The synthesis and work-up procedures were performed following the method described for compound **1-1**, and compound **7-1** (10.50 g, yield = 94%) was obtained. LCMS (ESI) m/z: 245.0 [M+H]⁺.

### Step 2: ethyl 2-(2-methoxy-4-((S)-3-(((R)-1-(naphthalen-1-yl)ethyl)amino)pyrrolidin-1-yl)phenyl)acetate (7-2)

To a 100 mL single-necked flask, the free compound **A** (1.47 g, 6.11 mmol), compound **7-1** (2.00 g, 7.32 mmol), Pd(OAc)₂ (70 mg, 0.31 mmol), X-Phos (150 mg, 0.31 mmol), Cs₂CO₃ (5.96 g, 12.50 mmol), and toluene (20 mL) were added. The synthesis and work-up procedures were carried out following the method described for compound **1-2**, and compound **7-2** (1.60 g, yield = 61%) was obtained. LCMS (ESI) m/z: 433.2 [M+H]⁺.

### Step 3: 2-(2-methoxy-4-((S)-3-(((R)-1-(naphthalen-1-yl)ethyl)amino)pyrrolidin-1-yl)phenyl)acetic acid (7)

To a 100 mL single-necked flask, compound **7-2** (1.60 g, 3.70 mmol), sodium hydroxide (0.59 g, 14.75 mmol), purified water (5 mL), and ethanol (5 mL) were added. The synthesis and work-up procedures were performed following the method described for compound **1**, and compound **7** (0.50 g, yield = 33%) was obtained. LCMS (ESI) m/z: 405.2 [M+H]⁺. ¹H NMR (600 MHz, DMSO-*d*₆) δ 8.33 (d, *J* = 8.3 Hz, 1H), 7.93 (d, *J* = 7.8 Hz, 1H), 7.81 (d, *J* = 8.0 Hz, 1H), 7.75 (d, *J* = 7.2 Hz, 1H), 7.51 (q, *J* = 7.9, 6.8 Hz, 3H), 6.88 (d, *J* = 8.2 Hz, 1H), 5.98 (d, *J* = 2.3 Hz, 1H), 5.96 (dd, *J* = 8.4, 2.2 Hz, 1H), 4.76 (s, 1H), 3.70 (s, 3H), 3.32 (s, 4H), 3.12 (dt, *J* = 9.2, 7.3 Hz, 1H), 2.99 (s, 1H), 2.04 (s, 1H), 1.91 (s, 1H), 1.42 (s, 3H).

### Example 9: Synthesis of 2-(2-ethoxy-4-((S)-3-(((R)-1-(naphthalen-1-yl)ethyl)amino)pyrrolidin-1-yl)phenyl)acetic acid (8)

The synthetic route is as follows:

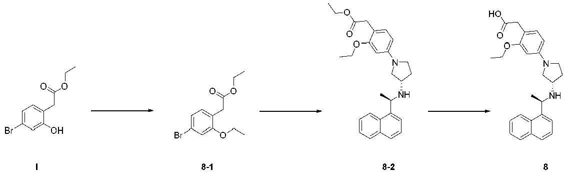

### Step 1: ethyl 2-(4-bromo-2-ethoxyphenyl)acetate (8-1)

To a 250 mL three-necked flask, compound **I** (2.00 g, 7.72 mmol), potassium carbonate (3.20 g, 23.00 mmol), bromoethane (0.93 g, 8.40 mmol), and acetonitrile (50 mL) were added. The reaction mixture was stirred at 60°C for 24.0 h. The reaction mixture was cooled to room temperature, and concentrated under reduced pressure until no solvent was evaporated. Ethyl acetate (50 mL) and purified water (50 mL) were added, and the mixture was stirred. The mixture was allowed to stand until obvious phase separation occurred, and the organic phase and the aqueous phase were separated. The organic phase was retained, and the aqueous phase was extracted once with 50 mL of ethyl acetate. The organic phases were combined and dried with anhydrous sodium sulfate, followed by filtration. The filtrate was concentrated under reduced pressure to a constant weight to obtain compound **8-1** (2.02 g, yield = 91%). LCMS (ESI) m/z: 287.0 [M+H]⁺.

### Step 2: ethyl 2-(2-ethoxy-4-((S)-3-(((R)-1-(naphthalen-1-yl)ethyl)amino)pyrrolidin-1-yl)phenyl)acetate (8-2)

To a 100 mL single-necked flask, the free compound **A** (1.00 g, 4.16 mmol), compound **8-1** (2.00 g, 7.32 mmol), Pd(OAc)₂ (70 mg, 0.31 mmol), X-Phos (150 mg, 0.31 mmol), Cs₂CO₃ (4.51 g, 13.90 mmol), and toluene (20 mL) were added. The synthesis and work-up procedures were performed following the method described for compound **1-2**, and compound **8-2** (1.60 g, yield = 86%) was obtained. LCMS (ESI) m/z: 447.3 [M+H]⁺.

### Step 3: 2-(2-ethoxy-4-((S)-3-(((R)-1-(naphthalen-1-yl)ethyl)amino)pyrrolidin-1-yl)phenyl)acetic acid (8)

To a 100 mL single-necked flask, compound **8-2** (1.60 g, 3.53 mmol), sodium hydroxide (0.60 g, 15.00 mmol), purified water (10 mL), and ethanol (10 mL) were added. The synthesis and work-up procedures were performed following the method described for compound **1**, and compound **8** (0.52 g, yield = 35%) was obtained. LCMS (ESI) m/z: 419.2 [M+H]⁺. ¹H NMR (600 MHz, DMSO-*d*₆) δ 11.70 (s, 1H), 10.23 (s, 1H), 9.78 (s, 1H), 8.39 (d, *J* = 8.5 Hz, 1H), 8.12 (s, 1H), 8.02 (t, *J* = 8.9 Hz, 2H), 7.64 (d, *J* = 7.5 Hz, 2H), 7.60 (t, *J* = 7.5 Hz, 1H), 6.93 (d, *J* = 8.0 Hz, 1H), 6.02 (d, *J* = 9.1 Hz, 2H), 5.42 (s, 1H), 3.96 (q, *J* = 7.0 Hz, 2H), 3.82 (s, 1H), 3.40 (t, *J* = 7.1 Hz, 3H), 3.15 (q, *J* = 8.1 Hz, 1H), 2.34 (s, 1H), 2.26 (s, 1H), 1.76 (s, 3H), 1.29 (t, *J* = 7.0 Hz, 3H).

### Example 10: Synthesis of 2-(2-isopropoxy-4-((S)-3-(((R)-1-(naphthalen-1-yl)ethyl)amino)pyrrolidin-1-yl)phenyl)acetic acid (9)

The synthetic route is as follows:

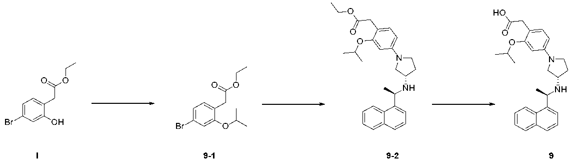

### Step 1: ethyl 2-(4-bromo-2-isopropoxyphenyl)acetate (9-1)

To a 250 mL three-necked flask, compound **I** (2.00 g, 7.72 mmol), potassium carbonate (3.20 g, 23.15 mmol), isopropyl bromide (1.04 g, 8.49 mmol), and acetonitrile (50 mL) were added. The synthesis and work-up procedures were carried out following the method described for compound **8-1**, and compound **9-1** (1.95 g, yield = 84%) was obtained. LCMS (ESI) m/z: 301.0 [M+H]⁺.

### Step 2: ethyl 2-(2-isopropoxy-4-((S)-3-(((R)-1-(naphthalen-1-yl)ethyl)amino)pyrrolidin-1-yl)phenyl)acetate (9-2)

To a 100 mL single-necked flask, the free compound **A** (0.80 g, 3.32 mmol), compound **9-1** (1.20 g, 3.32 mmol), Pd(OAc)₂ (40 mg, 0.16 mmol), X-Phos (80 mg, 0.16 mmol), Cs₂CO₃ (3.25 g, 9.96 mmol), and toluene (20 mL) were added. The synthesis and work-up procedures were carried out with reference to the method for compound **1-2**, and compound **9-2** (0.80 g, yield = 52%) was obtained. LCMS (ESI) m/z: 461.3 [M+H]⁺.

### Step 3: 2-(2-isopropoxy-4-((S)-3-(((R)-1-(naphthalen-1-yl)ethyl)amino)pyrrolidin-1-yl)phenyl)acetic acid (9)

To a 100 mL single-necked flask, compound **9-2** (0.80 g, 1.70 mmol), sodium hydroxide (0.20 g, 5.20 mmol), purified water (10 mL), and ethanol (10 mL) were added. The synthesis and work-up procedures were carried out with reference to compound **1**, and compound **9** (0.50 g, yield = 68%) was obtained. LCMS (ESI) m/z: 433.2 [M+H]⁺. ¹H NMR (600 MHz, DMSO-*d*₆) δ 8.32 (d, *J* = 8.0 Hz, 1H), 7.92 (dd, *J* = 7.2, 2.3 Hz, 1H), 7.79 (d, *J* = 8.1 Hz, 1H), 7.74 (d, *J* = 7.2 Hz, 1H), 7.51 (qd, *J* = 7.2, 5.1 Hz, 3H), 6.87 (d, *J* = 8.7 Hz, 1H), 5.94 (dq, *J* = 4.4, 2.3 Hz, 2H), 4.73 (d, *J* = 6.9 Hz, 1H), 4.50 - 4.42 (m, 1H), 3.29 (d, *J* = 4.1 Hz, 3H), 3.24 (t, *J* = 6.3 Hz, 1H), 3.20 (dd, *J* = 9.0, 6.6 Hz, 1H), 3.09 (dt, *J* = 9.1, 7.2 Hz, 1H), 2.92 (dd, *J* = 9.1, 5.5 Hz, 1H), 2.02 (dd, *J* = 12.2, 6.3 Hz, 1H), 1.87 (s, 1H), 1.40 (d, *J* = 6.6 Hz, 3H), 1.21 (d, *J* = 6.0 Hz, 6H).

### Example 11: Synthesis of 2-(4-((S)-3-(((R)-1-(naphthalen-1-yl)ethyl)amino)pyrrolidin-1-yl)-2-propoxyphenyl)acetic acid (10)

The synthetic route is as follows:

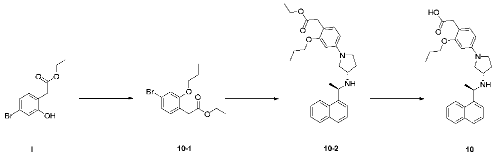

### Step 1: ethyl 2-(4-bromo-2-propoxyphenyl)acetate (10-1)

To a 250 mL three-necked flask, compound **I** (2.00 g, 7.72 mmol), potassium carbonate (3.20 g, 23.15 mmol), 1-bromopropane (1.04 g, 8.49 mmol), and acetonitrile (50 mL) were added. The synthesis and work-up procedures were carried out following the method described for compound **8-1**, yielding compound **10-1** (1.57 g, yield = 68%). LCMS (ESI) m/z: 301.0 [M+H]⁺.

### Step 2: ethyl 2-(4-((S)-3-(((R)-1-(naphthalen-1-yl)ethyl)amino)pyrrolidin-1-yl)-2-propoxyphenyl)acetate (10-2)

To a 100 mL single-necked flask, compound **A** in free form (1.04 g, 4.31 mmol), compound **10-1** (1.57 g, 5.20 mmol), Pd(OAc)₂ (50 mg, 0.22 mmol), X-Phos (100 mg, 0.22 mmol), Cs₂CO₃ (4.25 g, 13.04 mmol), and toluene (20 mL) were added. The synthesis and work-up procedures were performed following the method described for compound **1-2**, and compound **10-2** (0.45 g, yield = 23%) was obtained. LCMS (ESI) m/z: 461.3 [M+H]⁺.

### Step 3: 2-(4-((S)-3-(((R)-1-(naphthalen-1-yl)ethyl)amino)pyrrolidin-1-yl)-2-propoxyphenyl)acetic acid (10)

To a 100 mL single-necked flask, compound **10-2** (0.45 g, 0.98 mmol), sodium hydroxide (0.12 g, 3.00 mmol), purified water (10 mL), and ethanol (10 mL) were added. The synthesis and work-up procedures were performed following the method described for compound **1**, and compound **10** (0.38 g, yield = 90%) was obtained. LCMS (ESI) m/z: 433.2 [M+H]⁺. ¹H NMR (600 MHz, DMSO-*d*₆) δ 8.33 (d, *J* = 8.3 Hz, 1H), 7.94 (d, *J* = 7.8 Hz, 1H), 7.82 (s, 1H), 7.76 (s, 1H), 7.52 (dt, *J* = 15.4, 8.8 Hz, 3H), 6.88 (d, *J* = 8.3 Hz, 1H), 5.95 (s, 2H), 4.78 (s, 1H), 3.84 (t, *J* = 6.3 Hz, 2H), 3.32 (s, 4H), 3.27 (s, 1H), 3.11 (q, *J* = 7.8 Hz, 1H), 2.99 (s, 1H), 2.06 (s, 1H), 1.94 (s, 1H), 1.68 (h, *J* = 6.7 Hz, 2H), 1.44 (s, 3H), 0.97 (t, *J* = 7.4 Hz, 3H).

### Experimental Example 1: In Vitro Study of CaSR Conformational Activation Effect

The CaSR is a member of family C of G protein-coupled receptors (GPCR). Upon activation by extracellular Ca²⁺ or specific compounds, CaSR triggers an increase in intracellular Ca²⁺ concentration. To evaluate the effect of compounds on the allosteric activation of CaSR, a CHO cell line stably transfected with human CaSR (hCaSR-CHO) was utilized. Changes in intracellular Ca²⁺ concentration were measured as a key indicator to assess the conformational activation of CaSR.

### I. Experimental Procedure

1. Preparation Before Plate Spreading: Twelve hours prior to cell spreading, a 384-well cell culture plate was coated with 0.1 mg/mL polylysine.
2. Cell Spreading: Digested hCaSR-CHO cells were resuspended in complete medium, counted using a cell counter, and adjusted to a density of 5 × 10⁵ cells/mL. A volume of 25 µL of cell suspension was inoculated into each well. After inoculation, the cells were incubated at 37 °C in a 5% CO₂ incubator for 18 h.
3. Preparation of Compound Test Solution: The test compounds were diluted to the required concentrations (10 µM, 2 µM, 400 nM, 80 nM, 16 nM, 3.2 nM, and 0.64 nM) and added to a 384-well spiked plate. Since the cell culture plate already contained 25 µL of Fluo-4 NW assay reagent and the instrument was set to deliver 20 µL of the system, the compound concentrations in the spiked plate were 2.25 times the actual assay concentrations. For example, a 10 µM compound in the spiked plate corresponded to 22.5 µM in the assay wells.
4. Preparation of Assay Reagents: According to the instructions of the Fluo-4 NW Calcium Assay Kit (Thermo, F36206), 1 mL of assay buffer was added to Component B to prepare a 250 mM probenecid solution. 100 µL of the probenecid solution was added to 10 mL of assay buffer and mixed thoroughly. Component A was added to the mixture to prepare a 1X dye loading solution.
5. Fluorescent Dye Addition: The complete culture medium was discarded, and 25 µL of the 1X dye loading solution was added to the 384-well plate using a Multidrop automatic dispenser. The plate was incubated at 37 °C for 30 min in the dark, then transferred to room temperature for an additional 30 min. The plate was transferred to the FLIPER Tetra instrument (Molecular Devices).
6. Data Acquisition: After adding 20 µL of the compound test solution, the excitation wavelength was set to 470-495 nm, and the emission was detected at 515-575 nm. The instrument's quality control board was used to ensure no errors before initiating detection. The detection duration was 660 seconds, and the raw data were exported at the end of the reading.
7. Data Processing: The change in fluorescence intensity, representing intracellular calcium signals, was calculated by subtracting the minimum fluorescence value (average of 14-21 seconds) from the maximum fluorescence value (average of 40-80 seconds) after compound addition. Using GraphPad Prism 8.0, the EC₅₀ values of individual compounds were determined by plotting the compound concentration on the x-axis and the change in fluorescence intensity on the y-axis.

### II. Experimental Results

The *in vitro* pharmacodynamic results of selected compounds of the invention are summarized in Table 1.

**Table 1. Cellular pharmacodynamic data of the compounds.**

| Compounds | EC₅₀ (nM) |
|---|---|
| Evocalcet | 67.62 |
| 1 | 39.68 |
| 2 | 27.06 |
| 3 | 20.67 |
| 4 | 51.98 |
| 5 | 39.76 |
| 6 | 42.53 |
| 7 | 55.48 |

### Experimental Example 2: In Vivo Experiment to Inhibit PTH Concentration in Blood of Model Rats

Male SD rat models of hyperparathyroidism were constructed by five-sixths nephrectomy plus a special diet. Blood parathyroid hormone (PTH) level was used as the endpoint. ELISA was applied to determine the changes in plasma PTH concentration following compound treatment, thereby assessing the in vivo activity of the compound.

### I. Experimental Procedure

1. Model Construction: Specific pathogen-free (SPF)-grade male SD rats aged 7-8 weeks were used. The rats were acclimatized with normal feed for 1 week. Two-thirds of the left kidney was removed, and the rats were allowed to recover with normal feed for 1 week. Subsequently, the right kidney was removed, and the rats were allowed to recover with normal feed for an additional week. The rats were then fed a special diet (0.5% calcium and 1.2% total phosphorus) for 2 weeks.
2. Detection and Grouping: Approximately 300 µL of blood was collected from the tail vein into a coagulation-promoting tube, left at room temperature for 30 minutes, and centrifuged at 12,000 rpm for 3 minutes to obtain serum samples. The concentration of intact PTH (1-84) in the serum was detected using the MicroVue Intact PTH EIA-96 Test (Quidel, catalog No. 60-2500). The rats were grouped based on their PTH concentration levels, ensuring that the mean PTH concentration in each group was approximately the same.
3. Administration and Activity Testing: A single dose of the compound was administered by oral gavage. Blood samples were collected before and after administration, and serum was obtained using the method described in step 2. PTH levels were measured using ELISA. Using this method, it was confirmed that the compound effectively reduced PTH levels in the blood of the model rats.

### II. Experimental Results

The effects of selected compounds of the invention on reducing blood PTH levels in rat models are summarized in Table 2.

**Table 2. In vivo pharmacological effects of the compounds.**

| Compounds | The reduction efficiency (%) |
|---|---|
| vehicle | 0 |
| 2 | 91 |
| 3 | 93 |
| 5 | 81 |

| | |
|---|---|
| **Note:** The efficiency of PTH reduction at 1 hour post-administration was compared with the baseline PTH concentration at 0 hour. | |

### Experimental Example 3: Rat Liver Microsome Stability Test

Rat liver microsomes at a defined concentration, testosterone (positive control), tolbutamide (internal standard), the test compounds, and PBS (pH 7.4) were incubated at 37 °C. The reaction was quenched with methanol at 0, 5, 10, 20, 30, and 60 min, respectively. The samples were pretreated and analyzed using LC-MS. Data processing involved plotting the ratio of the peak area of the test compound to that of the internal standard (y-axis) against time (x-axis). The half-life (t_{1/2}) was calculated from the data. The stability test results for selected compounds of the invention are summarized in Table 3.

**Table 3. Stability data of compounds in rat liver microsomes.**

| Compounds | t_{1/2} (min) |
|---|---|
| Evocalcet | >60 |
| 2 | >60 |
| 3 | >60 |
| 5 | >60 |

| | |
|---|---|
| **Note:** The hepatic microsomal stability test was influenced by the activity of liver microsomes. In the *in vitro* test, the maximum incubation time was set at 60 minutes. A t_{1/2} > 60 minutes was considered to indicate high metabolic stability of the compound *in vivo*. | |

### Experimental Example 4: Pharmacokinetic Study in SD Rats

### I. Experimental Procedure

Animal Information: Male Sprague-Dawley (SD) rats, aged 7-8 weeks, were used. Each group consisted of 3-5 rats.

Dosing Information: The rats were fasted for more than 12 hours prior to administration. A single dose was administered via oral gavage.

Blood Sampling Time Points: Blood samples were collected from the tail vein before administration and at 0.25, 0.5, 1, 2, 4, 6, 8, and 24 hours post-administration. A volume of 100 µL of blood was collected at each time point.

Sample Processing and Storage: The blood samples were transferred into 1.5 mL microcentrifuge tubes, with 50 µL per tube (two tubes per sample). Centrifugation was completed within 2 hours. Whole blood samples were kept on ice prior to centrifugation. Centrifugation was performed at 4000 rpm, 2-8°C, for 10 minutes. After collecting the supernatant, it was stored at -80°C or below if not analyzed immediately.

### Detection:

### a) Chromatographic Conditions:

Column: An InfinityLab Poroshell 120 SB-C18 column (2.1 × 100 mm, 2.7 µm) was used. Column Temperature: The temperature was maintained at 30°C. Flow Rate: The flow rate was set at 0.3 mL/min. Run Time: The total run time was 9.5 minutes. Post-run Time: A post-run time of 2 minutes was applied. Injection Volume: An injection volume of 2 µL was used. Mobile Phase: A mixture of methanol and 0.1% formic acid was employed as the mobile phase. Gradient Elution: Gradient elution was performed according to the table below.

| Time (min) | Phase A (water with 0.1% formic acid) | Phase B (methanol) |
|---|---|---|
| 0 | 60 | 40 |
| 8.0 | 10 | 90 |
| 9.0 | 10 | 90 |
| 9.5 | 60 | 40 |

### b) The Mass Spectrometry Conditions Were Set As Follows:

| Projects | Values |
|---|---|
| Ion source | AJS ESI |
| Ion jet voltage | 3500 V |
| Gas temperature | 300 °C |
| Detection mode | Positive ion mode |
| Scan mode | Multi Reaction Monitoring (MRM) |
| Quantitative analysis of ion reactive | compounds m/z: (depending on the specific compound selection); Internal standard clenbuterol hydrochloride m/z 277.1 → 203 |
| Cluster voltage(V) | 50(2); 55(3) |
| Collision energy(V) | 30(2); 25(3) |

### c) Preparation of Stock Solutions and Working Solutions

All prepared stock solutions were stored at -80°C, while working solutions were stored at 4°C. Evocalcet Stock Solution (0.2 mg/mL): 10 mg of Evocalcet was accurately weighed and transferred into a 50 mL volumetric flask. The compound was dissolved in methanol and diluted to the mark, followed by thorough mixing to obtain the stock solution. (The preparation of stock solutions for other compounds followed the same procedure as for Evocalcet.)

Preparation of Standard Solutions: Appropriate volumes of Evocalcet, Compound 2, and Compound 3 stock solutions were precisely measured and diluted with 50% methanol to prepare standard solutions with concentrations of 80, 200, 1000, 5000, 10000, 20000, and 40000 ng/mL.

Preparation of Internal Standard (Clenbuterol Hydrochloride) Solution: 10 mg of clenbuterol hydrochloride was accurately weighed and transferred into a 10 mL volumetric flask. The compound was dissolved in methanol and diluted to the mark, followed by thorough mixing to obtain the clenbuterol hydrochloride stock solution (1.0 mg/mL). An appropriate volume of the stock solution was precisely measured and diluted with methanol to prepare a 2000 ng/mL internal standard working solution.

### d) Sample Processing Procedure

Standard Curve Sample Processing: 90 µL of blank matrix was aliquoted, and 10 µL of each standard series solution and 50 µL of internal standard solution were added. The mixture was vortexed for 30 seconds. Then, 350 µL of methanol was added to precipitate proteins, and the mixture was vortexed again. The samples were centrifuged at 12,000 rpm for 10 minutes. Subsequently, 200 µL of the supernatant was collected and mixed with 600 µL of water. The final supernatant was injected for analysis.

Blood Sample Processing: 50 µL of plasma sample was aliquoted, and 25 µL of internal standard solution was added. The mixture was vortexed for 30 seconds. Then, 175 µL of methanol was added to precipitate proteins, and the mixture was vortexed again. The samples were centrifuged at 12,000 rpm for 10 minutes. Subsequently, 100 µL of the supernatant was collected and mixed with 300 µL of water. The final supernatant was injected for analysis.

### II. Experimental Results

### The results are shown in Table 4.

**Table 4. Pharmacokinetic experiment results.**

| Compounds | Cₘₐₓ (ng/mL) | tₘₐₓ | t_{1/2} | AUC_{0-∞} (ng/mL) | Relative bioavailability (%) |
|---|---|---|---|---|---|
| Evocalcet | 2516.62±211.66 | 1.15±1.60 | 4.83±0.39 | 25434.64±7362.50 | 100 |
| 2 | 3545.48±282.74 | 0.65±0.34 | 5.91±0.21 | 34651.22±2710.64 | 136 |
| 3 | 3770.05±584.50 | 0.55±0.27 | 5.17±0.38 | 36633.75±1965.79 | 144 |

## Claims

1. A compound having a structure represented by formula (I), or a pharmaceutically acceptable salt, or a stereoisomer, or a tautomer thereof:
wherein R¹, R², R³, R⁴, and R⁵ are each independently selected from the group consisting of a hydrogen atom; C₂₋₆ carboxyl; C₁₋₇ alkoxy which may be optionally substituted with substituents selected from the group consisting of halogen, cyano, nitro, oxygen, C₁₋₆ carboxyl, amino, C₃₋₆ cycloalkyl, aryl C₁₋₆ alkoxy, hydroxyl, C₁₋₆ alkoxy, halogenated C₁₋₆ alkyl, and halogenated C₁₋₆ alkoxy; halogen; C₁₋₆ alkyl; and halogenated C₁₋₆ alkyl;
provided that 1) R¹, R², R³, R⁴, and R⁵ are not simultaneously H; and 2) when the substituent is the C₂₋₆ carboxyl, the substitution site is selected from R²-R⁴, and at least two substituents of R¹-R⁵ are not H.

2. The compound or the pharmaceutically acceptable salt, or the stereoisomer, or the tautomer thereof according to claim 1, wherein R¹ and R⁵ are each independently selected from H, halogen, C₁₋₆ alkyl, and C₁₋₆ alkoxy.

3. The compound or the pharmaceutically acceptable salt, or the stereoisomer, or the tautomer thereof according to claim 1 or 2, wherein at least one substituent of R²-R⁴ is an unsubstituted C₂₋₆ carboxyl or a substituted C₂₋₆ carboxyl, preferably carboxymethyl, and at least two substituents of R¹-R⁵ are not H; preferably, at least one substituent of R²-R⁴ is an unsubstituted carboxymethyl or a substituted carboxymethyl, and at least two substituents of R¹-R⁵ are not H.

4. The compound or the pharmaceutically acceptable salt, or the stereoisomer, or the tautomer thereof according to any one of claims 1 to 3, wherein R¹, R², R⁴, and R⁵ are each independently selected from H, halogen, C₁₋₆ alkyl, and C₁₋₆ alkoxy which may be optionally substituted with substituents selected from hydroxyl and C₁₋₆ alkoxy, and R¹, R², R⁴, and R⁵ are not simultaneously H; and R³ is C₂₋₆ carboxyl;
preferably, R¹, R², R⁴, and R⁵ are each independently selected from H, halogen, C₁₋₆ alkyl, and C₁₋₆ alkoxy which may be optionally substituted with substituents selected from hydroxyl and C₁₋₆ alkoxy, and R¹, R², R⁴, and R⁵ are not simultaneously H; and R³ is carboxymethyl.

5. The compound or the pharmaceutically acceptable salt, or the stereoisomer, or the tautomer according to any one of claims 1 to 4, wherein the compound of formula (I) is selected from:

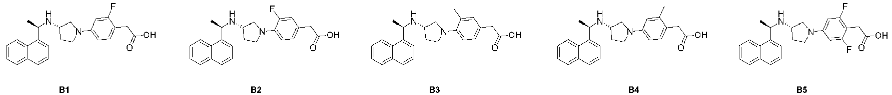

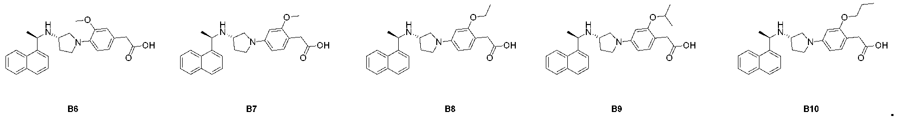

6. A method for preparing the compound of formula (I) according to any one of claims 1 to 5, comprising the step of reacting a compound of formula A with a compound of formula (II) to form the compound of formula (I): 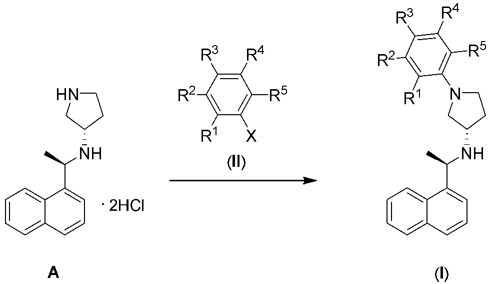 wherein X represents a leaving group selected from halogen, hydroxyl, lower alkylsulfonyloxy (e.g., C₁₋₆ alkylsulfonyloxy), sulfonyloxy, and the like, preferably selected from Br, Cl, or trifluoromethanesulfonyloxy.

7. A pharmaceutical composition comprising the compound, or the pharmaceutically acceptable salt, or the stereoisomer, or the tautomer thereof according to any one of claims 1 to 5, a pharmaceutically acceptable carrier or excipient.

8. Use of the compound, or the pharmaceutically acceptable salt, or the stereoisomer, or the tautomer thereof according to any one of claims 1 to 5, or the pharmaceutical composition according to claim 6, in the preparation of a medicament for preventing and/or treating diseases related to CaSR.

9. The use according to claim 8, wherein the disease is selected from primary hyperparathyroidism, secondary hyperparathyroidism, tertiary hyperparathyroidism, chronic renal failure, chronic kidney disease, parathyroid adenoma, parathyroid hyperplasia, parathyroid carcinoma, vascular calcification and valvular calcification, abnormal calcium homeostasis, abnormal phosphorus homeostasis, bone-related diseases or complications caused by hyperparathyroidism, chronic kidney disease, or parathyroid carcinoma, bone loss after kidney transplantation, osteitis fibrosa cystica, adynamic bone disease, renal osteodystrophy, cardiovascular complications caused by hyperparathyroidism or chronic kidney disease, and certain malignancies with abnormally high Ca²⁺ levels.
